Europäisches Patentamt

European Patent Office

Office européen des brevets

(11) Publication number: **0 124 044**
**B1**

(12) EUROPEAN PATENT SPECIFICATION

(45) Date of publication of patent specification: 05.09.90

(51) Int. Cl.⁵: **A 61 L 2/04**

(21) Application number: **84104493.6**

(22) Date of filing: **19.04.84**

(54) Process for pasteurizing fibronectin.

(30) Priority: 29.04.83 US 489886

(43) Date of publication of application:
07.11.84 Bulletin 84/45

(45) Publication of the grant of the patent:
05.09.90 Bulletin 90/36

(84) Designated Contracting States:
AT BE CH DE FR GB IT LI LU NL SE

(56) References cited:
EP-A-0 035 204
EP-A-0 050 061
EP-A-0 052 827
EP-A-0 099 445
FR-A-2 532 178

(73) Proprietor: Armour Pharmaceutical Company
303 South Broadway
Tarrytown New York 10591 (US)

(72) Inventor: Landaburu, Ricardo H.
1, Magnolia Drive
Rye Town New York (US)
Inventor: Amphlett, Godfrey
48 Hutchinson Blvd
Mount Vernon New York (US)
Inventor: Branson, Roy E.
174 Grat Plain Road
Danbury Connecticut (US)
Inventor: Shaw, Arthur B.
6 Alexander Avenue
Harrison New York (US)

(74) Representative: Patentanwälte Grünecker,
Kinkeldey, Stockmair & Partner
Maximilianstrasse 58
D-8000 München 22 (DE)

Courier Press, Leamington Spa, England.

**Description**

This invention relates to a process for heat treatment of a solution containing the protein fibronectin and a polyol to inactivate viruses therein while preserving the protein's native character. By the process according to the present invention fibronectin is rendered free of transmissible biological contaminants such as viruses, and especially infectious hepatitis viruses, such as hepatitis virus B, and the Non-A, Non-B infectious virus(es).

Fibronectin is a large complex protein possessing important biological functions such as acting as a structural component of tissues and modulating the function of cells in the body. Its actions include control of normal growth, participation in the repair of damage, and involvement in defense against invading foreign substances and organisms. It is known by a variety of names that reflect its diverse biological activities including the names of: large external transformation sensitive protein (LETS); cell surface protein (CSP); cell adhesion protein (CAP); opsonic α2 surface binding glycoprotein; and cold insoluble globulin (CIG). Pharmacological application of fibronectin includes treatment of septic shock and infective diseases. Due to its action of enhancing intercellular adhesive properties and its effect on the morphology of cancer cells, it is a potential candidate for cancer treatment.

Fibronectin is obtained from fractions of plasma protein or fibroblast culture fluid. Contamination by viruses may result from both the source material used and from the environment during the separation procedure.

Although efforts are being made to utilize plasma which is negative to hepatitis virus B when tested using high sensitivity methods such as radioimmunoassay or passive hemagglutination, the danger of such contamination still exists because the presence of the virus cannot be detected with certainty by any known procedure. In addition, there are no available *in vitro* test methods to detect the Non-A, Non-B, Hepatitis virus(es).

To be suitable for clinical application, fibronectin must be free of contaminants, especially infectious hepatitis viruses, it must possess a high degree of therapeutic activity, and it must have a good shelf-life.

It is known that plasma protein may be rendered free of infectious hepatitis viruses by deactivation with heat treatment for 16 hrs at about 60°C. The problem confronting the prior art is the thermal instability of fibronectin: heat treatment above 40—45°C causes coagulation, denaturation and loss of activity therein.

It has been known for many years that certain polyols confer heat stability to proteins against denaturation during pasteurization sufficient to render hepatitis virus inactive. Such polyols include polyhydric alcohols and carbohydrates, such as arabinose, glucose, galactose, fructose, ribose, mannose, rhamnose, sucrose, maltose and raffinose.

Fibronectin has also been stabilized during or prior to pasteurization by certain sugars such as sucrose, and by the use of certain sugars in conjunction with some other stabilizing agents, such as neutral amino acids, hydrocarbon carboxylic acids, and hydroxyhydrocarbon carboxylic acids.

While inactivation of infectious hepatitis viruses has been adequately attained and heat stability of proteins in general was markedly increased, the native character of such proteins has not been sufficiently preserved from denaturation. This lack of complete preservation of physical and biological activity is especially apparent in large scale production of proteins such as fibronectin. As opposed to the mechanically static conditions of laboratory scale procedures wherein, as a result of large surface area to small volume ratio, effective transfer of heat into the solution is easily attained without mechanical agitation, in large scale manufacturing procedures the surface to volume ratio of the liquid being processed is drastically reduced and extensive mechanical agitation is necessary to effect uniform temperature therein. It has been observed that when heat and mechanical agitation are applied stimulaneously during pasteurization, carbohydrates will not fully protect fibronectin from denaturation and loss of yield It has also been found that in large scale manufacturing operations, in addition to the denaturing forces of heat and mechanical stress, polymeric forms of fibronectin are generated as a consequence of the catalyzing action of certain metal ions which may be present in the solution being processed or in the sterilizing equipment used from which they leach out into the solution from such objects as vessel walls, valves and surfaces of the operating equipment of metal and nonmetal construction. An example of such an undesirable metal ion is $Cu^{2+}$ which causes polymerization of fibronectin via disulfide formation through a free sulfhydryl group in the fibronectin molecule. The presence of even a minute amount of metal ions is sufficient to cause the undesired polymerization of fibronectin during the prolonged heating and agitation.

EP—A—35 204 discloses a method for pasteurization of approximately 20 different therapeutically active proteins in compositions containing the therapeutically active protein, for example, fibronectin and adducts. The procedure according to EP—A—35 204 requires the addition of at least polyol. The stabilizing effect for fibronection, however, could not clearly be demonstrated.

Therefore, it was the object of the present invention to provide a reliable and practical process for the heat treatment of a solution containing fibronectin and a polyol, in order to inactivate viruses therein, while preserving the protein's native character.

This object has been achieved by a process, as mentioned above, wherein a pharmaceutically acceptable surface active agent is present in an amount of about 0.01% to 0.5% w/v and a pharmaceutically acceptable chelating agent is present in amounts sufficient to confer stability to the protein and preserve the native character of said protein and the heat treatment is carried out at a temperature of about 50°C to 70°C.

In the prior art there are procedures known for the treatment of solutions containing pharmaceutically active proteins in order to minimize the potential risks for patients. However, these procedures do not resemble pasteurization procedures because they are not performed at temperatures above 37°C. It should be emphasized that classical pasteurization requires temperatures between 60°C and 80°C.

For example, EP—A—50 061 discloses a process for the suppression or reduction of undesirable activities such as pyrogenicity, hepatitis infectivity and clotting activation which includes the treatment of biological or pharmaceutical products by a prolonged contact with a non-denaturing amphiphile. This procedure is not conducted at elevated temperatures but at 4°C to 37°C. The conditions described in EP—A—50 061 do not result in a complete loss of infectivity of hepatitis viruses but merely lead to a substantial reduction of infectivity (page 4 of said application, lines 10 and 11) which is not supported by data. Moreover, the procedure according to EP—A—50 061 requires an enormous amount of surface active agents, namely from about 0.25% to 10% by weight, preferably 2% to 3% (claim 8), in contrast to the procedure according to the present invention which requires a maximum of 0.5% surfactant. It is well known to those skilled in the art that surface active agents have to be removed prior to the use of the sterilizing sample as a therapeutically active substrate. Therefore, a less high percentage of surface active agent is a desired improvement.

FR—A—25 32 187 discloses a procedure for the sterilization of tumor necrosis factor (TNF) by the addition of polyol and a non-ionic surface active agent. This invention only deals with the sterilization of a certain protein at 4°C and does not describe a procedure for the pasteurization or sterilization under elevated temperatures.

Use of polyols and chelating agents for sterilization of pharmaceutically active proteins is already known for preparations containing clotting factors II and VII, as described in EP—A—52 827. These proteins, however, are not prone to polymerization and possess entirely different properties and functions than fibronectin. Therefore, the sterilization of these proteins does not involve the problem of polymerization, which arises with any treatment concerning a fibronectin-containing solution.

According to the present invention, it has been found that the native character and biological properties of fibronectin may be preserved during an effective pasteurization procedure against viruses and without the formation of undesirable polymeric forms of fibronectin by utilizing in the aqueous solution containing fibronectin: a heat stability conferring polyol; a surface active agent in an amount of about 0.01% to 0.5% w/v which by the properties conferred on the solvent and its interaction with fibronectin diminishes the mechanical denaturing stresses resulting from agitation; and a chelating agent in amounts sufficient to confer stability to the protein to sequester and render inactive, polymerization catalyzing trace metals present in the fibronectin solution or in the sterilizing apparatus.

Thus the present invention encompasses the process of heat treatment to inactivate viruses of an aqueous solution containing fibronectin comprising: carrying out heat treatment effective to pasteurize the aqueous solution containing fibronectin, such as heat treatment at about 60°C for about 10 hours in the presence of about 25% to 60% w/v of a polyol, about 0.01% to 0.5% w/v of a pharmaceutically acceptable surface active agent, and about 0.0005 to 0.2 M of a pharmaceutically acceptable chelating agent.

In general, to the aqueous solution containing fibronectin, appropriate amounts of polyol, surface active and chelating agents are added forming a suspension which is then heated for 10 hrs at 60°C. After pasteurization the viscous suspension is diluted and filtered through ion exchange columns or ligand-affinity columns to remove the additives and non-specifically bound proteins. The pure fibronectin is then formulated into appropriate dosages.

In accordance with the present invention, fibronectin is pasteurized in the presence of a polyol, a surface active agent and a chelating agent, the combined effect of which insures that fibronectin is preserved in its native form during pasteurization.

The heat treatment is carried out at a temperature and for a period of time which are sufficient to inactivate the viruses, especially infectious viruses such as hepatitis viruses, but at the same time to retain the activity of fibronectin. Such heat treatment may be for 5—20 hrs, preferably for about 10 hours at 50°—70°C, and most preferably at 60°C for 10 hrs.

For the purposes of the present invention, in addition to using a purified fibronectin, various starting solutions of fibronectin may be used, including impure forms of fibronectin such as human plasma, cyroprecipitate, the acid-chill precipitate obtained from cryoprecipitate which is analogous to Fraction I-O, or Fraction I. While, as indicated, the aqueous solution containing fibronectin to be used as starting material in the present invention may be in any stage of purification ranging from purified to unpurified aqueous solution, we prefer to use at least a partially purified aqueous fibronectin.

Polyols

In the present invention we prefer to use the disaccharide sucrose (saccharose) as the preferred agent for conferring heat stability to fibronectin during pasteurization. We have found that a concentration range of about 25 to 60% w/v is effective for such stabilization, with the preferred concentration being about 30% to 50% w/v. While on the one hand, even lower concentrations might be used for the stabilization, the yield of fibronectin tends to be affected at such lower concentration, and on the other hand, higher than the stated concentration limit will deleteriously affect subsequent processing of the pasteurized solution due to the high viscosity obtained by the large amounts of sugar present.

Other polyols may be substituted for the disaccharide sucrose, such as: other disaccharides, such as maltose and lactose; monosaccharides, such as glucose, galactose and mannose; certain polyhydric alcohols, such as glycerol; and certain polymers such as polyethylene glycol.

## Surface active agents

Surface active agents used to advantage in accordance with the present invention must have the criteria of pharmaceutical acceptability and capability of easy removal from the solution after pasteurization thereof. A small quantity of these surface active agents was found to be effective to provide stability to fibronectin when used in conjunction with a polyol which is unable to prevent at least a partial loss due to denaturation and polymerization of fibronectin due to the combination of heat treatment and mechanical agitation. It was found that the use of pharmaceutically acceptable surface active agents in a concentration range of 0.01% to 0.5% w/v is sufficient to lend the protective effect to the fibronectin, while a concentration range of about 0.02% to 0.10% w/v is preferred. It was further found that even though higher quantities of particular surface active agents would perform satisfactorily, the use of excessive amounts of same is undesirable for reason of difficulty encountered with removal thereof from the pasteurized solution.

It was found that poly(oxy-alkylene) mono- and trisorbitan esters (fatty acid esters of sorbitol and its anhydrides copolymerized with a varying number of moles of ethylene oxide), are well suited as surface active agents for the purposes of the present invention, such as Polysorbate 80 (an oleate ester), Polysorbate 20 (a laurate ester), Polysorbate 40 (a palmitate ester), and Polysorbate 60 (a stearate ester).

Other surface active agents useful in the present invention include nonionic agents such as alkyl phenyl polyoxyenthylenes (such as Triton® and Nonidet®); anionic agents such as the bile salts (sodium taurocholate, sodium cholate, sodium deoxycholate and sodium glycocholate); cationic agents such as benzoalkonium chloride; and polyhydric alcohols with surface active properties such as the high molecular weight copolymers of propylene glycol and propylene oxide sold under the trade names of Pluronic[R] F-38 and Pluronic[R] F-68.

## Chelating agents

As previously indicated, in addition to the denaturing forces of heat and mechanical stress, a catalytic chemical action can contribute to the denaturation of the protein being sterilized. Heat and agitation tend to accelerate such catalytic chemical action resulting in the formation of polymeric forms of fibronectin. Trace quantities of certain metal ions, such as $Cu^{2+}$, are sufficient to cause such an undesired result. Other metal ions known to be reactive with the sulfyhydryl moieties of proteins may cause similar activity or simply form insoluble sulfide salts. Whereas these metal ions may not per se be present in the solution of protein being pasteurized, such various surfaces of the pasteurizing apparatus. To sequester these metal ions a chelating agent is used in the range of about 0.0005 to 0.2 molar preferably in the range of about 0.001 to .01 molar, such as ethylenediaminetetraacetic acid (EDTA), ethylene-bis-oxyethylenenitrile (EGTA), and orthophenanthrolene. Other chelating agents which are easily separated from the solution upon completion of pasteurization are also in the purview of the present invention.

In combination with chelating agents, a buffer solution may also be employed to sequester trace metal ions, such as a sodium citrate buffer in 0.0005 to 0.2 molar concentration range.

An alternative method for preventing the oxidation of the sulfhydryls may be the addition of 0.0005 to 0.2 molar of directly-acting antioxidants, such as ascorbic acid or cysteine.

The invention will be illustrated in more detail with reference to the following examples.

## Example 1

5 kg Fraction I-O or its equivalent derived from cryoprecipitate was resuspended and dissolved in 3 volumes of sodium chloride (0.15 M), buffered with sodium citrate (0.01 M) and glycine (0.05 M) (hereinafter cgs buffer) at pH 7.3, by warming the solution at 37°C with gentle stirring. The solution was clarified by ordinary filtration on non-fiber releasing media such as Zetapor® (AMF Cuno) with a diatomaceous earth filter-aid coating. The resultant solution was diluted with approximately 20 volumes of the same buffer with concurrent addition of sufficient sucrose to make the final solution 50% w/v, Polysorbate 80 was added to obtain a concentration of 0.05% w/v followed by the addition of sufficient EDTA to make the final solution 0.005 M.

With vigorous mechanical agitation the solution of fibronectin in the impure form and comprising 115 liters was heated in a stainless steel tank of 0.189 m³ (50 gallon) capacity with an integral heat exchange surface so as to achieve a temperature of 60°C in a period of one to four hours. On achieving an internally recorded solution temperature of 59.5°C, the solution was held with agitation continued for 10 hours at 60±0.5°C. It was then diluted with a chilled pasteurization medium, from which sucrose has been omitted, to a volume of about 230 liters, so as to effect the lowering of temperature to about 20—45°C. At this stage a considerable precipitate in the form of denatured protein, which does not include fibronectin, will have formed as the result of the prolonged heating, and this was removed from the solution by ordinary filtration on media such as Zetapor® using a diatomaceous earth filter aid.

The filtrate from this process was retained at a temperature of about 20°C to 40°C until further purification was effected.

Example 2

Ten liters of human plasma, from which antihemophilic factor has been removed without loss of the majority of the fibronectin present in the native plasma, was adjusted to contain 50% sucrose (w/v) of final solution in a final volume of 20 liters. The solution was made 0.01 M in sodium citrate, 0.05 M in glycine, and 0.005 M in EDTA without adjustment to the anticoagulant present during plasma collection. Polysorbate 80 was added to obtain a final concentration of 0.05% w/v. The pH was adjusted to between 7.0 and 7.4. The resultant solution was heated with vigorous agitation in a stainless steel vessel of 0.057 m³ (15 gallon) capacity, which contains an integral heat exchanger surface, so that a temperature of 60°C±0.5°C was reached in one to four hours. With continuous agitation, this temperature was maintained for a period of 10 hours. This solution was then diluted two-fold with chilled pasteurization medium from which sucrose has been omitted so as to effect lowering the temperature to about 20—45°C. Denatured protein which does not include the desired fibronectin remains in suspension, and this is removed by ordinary filtration on media such as Zetapor® using a diatomaceous earth filter aid.

Example 3

5 kg of Acid-Chill Precipitate obtained from human plasma is resuspended and dissolved in 3 volumes of cgs buffer at pH 7.3, by warming the solution at 37°C with gentle stirring. The solution was clarified by ordinary filtration on non-fiber releasing media such as Zetapor® (AMF Cuno) with a diatomaceous earth filter-aid coating. The resultant solution was diluted with approximately 20 volumes of the same buffer with concurrent addition of sufficient sucrose to make the final solution 50% w/v, Polysorbat 80 was added to obtain a concentration of 0.05% w/v followed by the addition of sufficient EDTA to make the final solution 0.005 M.

With vigorous mechanical agitation the solution of fibronectin in the impure form and comprising 80 liters was heated in a stainless steel tank of 0.095 m³ (25 gallon) capacity with an integral heat exchange surface so as to achieve a temperature of 60°C in a period of one to four hours. On achieving an internally recorded solution temperature of 59.5°C, the solution was held with agitation continued for 10 hours at 60±0.5°C. It was then diluted with a chilled pasteurization medium, from which sucrose has been omitted, to a volume of about 160 liters, so as to effect the lowering of temperature to about 20—45°C. At this stage a considerable precipitate in the form of denatured protein, which does not include fibronectin, will have formed as the result of the prolonged heating, and this is removed from the solution by ordinary filtration on media such as Zetapor® using a diatomaceous earth filter aid.

The filtrate from this process was cooled to 5°C (±2°C) and retained at this temperature until further purification was effected.

Next, the filtrate is warmed to 22°C and then applied at a flow rate of 20 ml/cm$_2$/hr to a Sepharose® gelatin column that has been previously prepared by the cyanogen bromide activation method, followed by washing with deionized 8 M urea in cgs buffer. Then the column containing fibronectin bound to the Sepharose® gelatin was washed with 1 M deionized urea in cgs buffer. In this step EDTA, Tween 80[R], sucrose and non-specifically bound proteins are removed.

Next, the fibronectin was eluted from the Sepharose® gelatin with a solution of 8 M urea in cgs buffer. The urea was removed from the fibronectin by passing the eluted fibronectin through a G25 Sephadex® column that was previously equilibrated with a solution containing 0.05 M NaCl and 2 mM sodium citrate at a pH of 7.3.

The so obtained fibronectin solution was made into formulations and sterile filtered.

A set of experiments using purified, unpasteurized fibronectin was performed to examine the effects of heat and agitation for varying periods of time on the properties of fibronectin. The experiments and results thereof are described in the examples that follow.

Example 4

This example demonstrates the agitation-induced precipitation effect in fibronectin. Aqueous buffered solutions of fibronectin (1 mg/ml) having adducts indicated below were agitated on a wrist action shaker at 25°C for 30 minutes. A fibrous white precipitate was visible in the samples denoted with (+). The protein lost from the solution was assayed by measuring the optical density of the remaining clear solution at 280 nm and comparing it with a control.

| Additions to 1 mg/ml fibronectin solution (w/v) | Formation of fibrous white precipitate | Percent protein lost |
|---|---|---|
| None | + | 1 |
| 50% Sucrose | + | 46 |
| 0.05% Tween 80® | – | 0 |
| 50% Sucrose+0.05% Tween 80® | – | 0 |

Example 5

This example demonstrates the heat-induced denaturation effect.

Aqueous buffered solution of fibronectin (1 mg/ml) having adducts indicated below were heated without agitation for 30 minutes at 60°C. Following dialysis against buffer, the samples were tested as follows.

a) Gelatin Binding Activity (assayed by a method analogous to that of Engvall et al., J. Exp. Med. *147*, 1584 (1978)).

| Additions to 1 mg/ml fibronectin solution (w/v) | Percent loss in gelatin binding activity compared with unheated control |
|---|---|
| None | 36.5 |
| 50% Sucrose | 18.7 |
| 0.05% Tween 80® | 25.3 |
| 50% Sucrose+0.05% Tween 80® | 0 |

b) Aggregates Detected by SDS Gel Electrophoresis.

| Additions to 1 mg/ml fibronectin solution (w/v) | Percent of protein with molecular weight greater then 440,000 daltons |
|---|---|
| None | 53 |
| 50% Sucrose | 15 |
| 0.05% Tween 80® | 63 |
| 50% Sucrose+0.05% Tween 80® | 15 |
| Unheated control | 19 |

c) Fluorescence Emission spectrum.

| Additions to 1 mg/ml fibronectin solutions (w/v) | Shift in emission spectrum relative to unheated control, nm |
|---|---|
| None | +3.5 |
| 50% Sucrose | 0 |
| 0.05% Tween 80® | +4.5 |
| 50% Sucrose+0.05% Tween 80® | 0 |

### Example 6

This example demonstrates both the heat induced denaturation and agitation-induced precipitation effects on fibronectin.

The procedure of Example 4 was repeated as described therein, except the agitation continued for 10 hrs at 58°—60°C.

| Additions to 1 mg/ml fibronectin solution (w/v) | Formation of fibrous white precipitate | Percent protein lost | Percent loss in gelatin binding compared with unheated control |
|---|---|---|---|
| 50% sucrose | + | 36 | 36 |
| 0.05% Tween 80® | − | 0 | 100 |
| 50% sucrose+0.05 Tween 80® | − | 0 | 0 |

As can be easily ascertained from the above examples the combination of a sugar and surfactant is necessary to prevent both the agitation-induced precipitation effect and the heat-induced denaturation effect when the fibronectin solution is pasteurized by heat and agitation; neither a sugar nor a surfactant alone prevents both effects.

### Example 7

This example demonstrates the metal ion-induced aggregation of fibronectin. Solutions of fibronectin (5 mg/ml in cgs buffer) having adducts indicated below were dialyzed against buffered solutions (0.05 M Tris-HCl, 0.1 M NaCl, pH 7.4) containing the same adducts for 24 hours at 25°C. The extent of aggregation was measured by SDS polyacrylamide gel electrophoresis.

| Additions to fibronectin and dialysis buffer | Percent of protein on SDS polyacrylamide gel with molecular weight greater than 440,000 daltons |
|---|---|
| None | 5 |
| 0.1 mM $CuCl_2$ | 20 |
| 0.1 mM $CuCl_2$+5 mM EDTA | 2 |

While the present invention has been described with reference to fibronectin, the pasteurizing/stabilizing procedures are also applicable to proteins other than fibronectin behaving similarly to fibronectin.

### Claims

1. A process for heat treatment of a solution containing fibronectin and a polyol to inactivate viruses therein while preserving the protein's native character, characterized in that a pharmaceutically acceptable surface active agent is present in an amount of 0.01% to 0.5% w/v and a pharmaceutically acceptable chelating agent is present in amounts sufficient to confer stability to the protein and preserve the native character of said protein and the heat treatment is carried out at a temperature of 50°C to 70°C.

2. The process of claim 1 wherein the polyol is sucrose, maltose, lactose, glucose, mannose, galactose or glycerol.

3. The process of any of claims 1 to 2 wherein the surface active agent is polyoxyethylene sorbitan mono- and tri-esters, alkyl phenyl polyoxyethylenes, bile salts, benzalkonium chloride or polyhydric alcohols.

4. The process of claim 3 wherein the polyoxyethylene sorbitan esters contain the fatty acids oleate, stearate, laurate, or palmitate.

5. The process of claim 3 wherein bile salt is sodium taurocholate, sodium cholate, sodium deoxycholate or sodium glycocholate.

6. The process of any of claims 1 to 5 wherein the chelating agent is ethylenediaminetetraacetic acid, ethylene-bis-oxyethylenenitrile or orthophenanthrolene.

7. The process of any of claims 1 to 6 wherein the protein solution further comprises a sodium citrate buffer.

8. The process of claim 7 wherein the concentration of the buffer is about 0.0005 to 0.2 molar.

9. The process of any of claims 1 to 8 wherein the polyol is present in an amount about 25% to 60% w/v.

10. The process of any of claims 1 to 9 wherein the chelating agent is present in an amount of about 0.0005 to 0.2 M.

11. The process of any of claims 1 to 10 wherein the heat treatment is carried out for 5 to 20 hours.

12. The process of claim 1 wherein the fibronectin solution is heat treated to effect pasteurization thereof in the presence of: 30% to 50% w/v of sucrose, maltose, lactose, glucose, mannose, galactose or glycerol; 0.02% to 0.1% w/v of polyoxy ethylene sorbitan mono- and tri-esters, benzalkonium chloride, sodium cholate, sodium taurocholate, sodium deoxycholate or sodium glycocholate; and 0.001 to 0.005 molar of ethylenediaminetetraacetic acid, citric acid, ethylene-bis-oxyethylenenitrile or orthophenanthrolene.

## Patentansprüche

1. Verfahren zum Wärmebehandeln einer Fibronectin und ein Polyol enthaltenden Lösung, um Viren darin zu inaktivieren, während der native Charakter des Proteins erhalten bleibt, dadurch gekennzeichnet, daß ein pharmazeutisch verträgliches oberflächenaktives Mittel in einer Menge von 0,01%—0,5% Gewicht/Volumen anwesend ist und ein pharmazeutisch verträgliches Chelat-bildendes Mittel in Mengen anwesend ist, die ausreichen, dem Protein Stabilität zu verleihen und den nativen Charakter des Proteins zu erhalten, und daß die Wärmebehandlung bei einer Temperatur von 50°C—70°C durchgeführt wird.

2. Verfahren nach Anspruch 1, dadurch gekennzeichnet, daß das Polyol Saccharose, Maltose, Lactose, Glucose, Mannose, Galactose oder Glycerin ist.

3. Verfahren nach einem der Ansprüche 1 bis 2, dadurch gekennzeichnet, daß das oberflächenaktive Mittel Polyoxyethylensorbitanmono- und tri-Ester, Alkylphenylpolyoxyethylene, Gallensalze, Benzalkoniumchlorid oder Polyalkohole sind.

4. Verfahren nach Anspruch 3, dadurch gekennzeichnet, daß die Polyoxyethylensorbitanester die Fettsäuren Oleat, Stearat, Laurat oder Palmitat enthalten.

5. Verfahren nach Anspruch 3, wobei das Gallensalz Natriumtaurocholat, Natriumcholat, Natriumdesoxycholat oder Natriumglycocholat ist.

6. Verfahren nach einem der Ansprüche 1 bis 5, dadurch gekennzeichnet, daß das Chelat-bildende Mittel Ethylendiamintetaressigsäure, Ethylen-bis-oxyethylennitril oder Orthophenanthrolin ist.

7. Verfahren nach einem der Anprüche 1 bis 6, dadurch gekennzeichnet, daß die Proteinlösung weiter einen Natriumcitratpuffer enthält.

8. Verfahren nach Anspruch 7, dadurch gekennzeichnet, daß die Konzentration des Puffers ungefähr 0,0005—0,2 Molar beträgt.

9. Verfahren nach einem der Ansprüche 1 bis 8, dadurch gekennzeichnet, daß das Polyol in einer Menge von ungefähr 25%—60% Gewicht/Volumen anwesend ist.

10. Verfahren nach einem der Ansprüche 1 bis 9, dadurch gekennzeichnet, daß das Chelat-bildende Mittel in einer Menge von ungefähr 0,0005—0,2 M anwesend ist.

11. Verfahren nach einem der Ansprüche 1 bis 10, dadurch gekennzeichnet, daß die Wärmebehandlung 5 bis 20 Stunden durchgeführt wird.

12. Verfahren nach Anspruch 1, dadurch gekennzeichnet, daß die Fibronectin-Lösung in Gegenwart von: 30%—50% Gewicht/Volumen Saccharose, Maltose, Lactose, Glucose, Mannose, Galactose oder Glycerin; 0,02%—0,1% Gewicht/Volumen Polyoxyethylensorbitanmono- und tri-Ester, Benzalkoniumchlorid, Natriumcholat, Natriumtaurocholat, Natriumdesoxycholat oder Natriumglychocholat; und 0,001—0,005 Molar Ethylendiamintetraessigsäure, Zitronensäure, Ethylen-bis-oxyethylennitril oder Orthophenanthrolin wärmebehandelt wird, um deren Pasteurisation zu bewirken.

## Revendications

1. Procédé de traitement thermique d'une solution contenant de la fibronectine et un polyol pour inactiver des virus contenus dans ladite solution, tout en préservant le caractère natif de la protéine, caractérisé en ce qu'un agent tensio-actif pharmaceutiquement tolérable est présent à raison de 0,01% à 0,5% en poids/volume et un agent de chélation pharmaceutiquement tolérable est présent en quantité suffisante pour procurer la stabilité à la protéine et pour préserver la caractère natif de ladite protéine et le traitement thermique est réalisé à une température comprise entre 50 et 70°C.

2. Procédé selon la revendication 1, dans lequel le polyol est du sucrose, du maltose, du lactose, du glucose, du mannose, du galactose ou du glycérol.

3. Procédé selon l'une des revendications 1 et 2, dans lequel l'agent tensio-actif consiste en des mono- et tri-esters de polyoxyéthylène sorbitane, des polyoxyéthylènes d'alkyl phényle, des sels biliaires, du chlorure de benzalkonium ou des alcools polyvalents.

4. Procédé selon la revendication 3, dans lequel les esters de polyoxyéthylène sorbitane contiennent les acides gras oléate, stéarate, laurate ou palmitate.

8

5. Procédé selon la revendication 3, dans lequel le sel biliaire est le taurocholate de sodium, le cholate de sodium, le désoxycholate de sodium ou le glycocholate de sodium.

6. Procédé selon l'une quelconque des revendications 1 à 5, dans lequel l'agent de chélation est l'acide éthylène-diamine-tétra-acétique, l'éthylène-bis-oxyéthylène-nitrile ou l'orthophénanthrolène.

7. Procédé selon l'une quelconque des revendications 1 à 6, dans lequel la solution protéique comprend en outre un tampon de citrate de sodium.

8. Procédé selon la revendication 7, dans lequel la concentration du tampon est d'environ 0,0005 à 0,2 mole.

9. Procédé selon l'une quelconque des revendications 1 à 8, dans lequel le polyol est présent à raison d'environ 25 à 60% p/v.

10. Procédé selon l'une quelconque des revendications 1 à 9, dans lequel l'agent de chélation est présent à raison d'environ 0,0005 à 0,2 M.

11. Procédé selon l'une quelconque des revendications 1 à 10, dans lequel le traitement thermique est réalisé pendant 5 à 20 heures.

12. Procédé selon la revendication 1, dans lequel la solution de fibronectine est traitée thermiquement pour réaliser la pasteurisation de celle-ci en présence de: 30% à 50% p/v de sucrose, de maltose, de lactose, de glucose, de mannose, de galactose ou de glycérol; 0,02% à 0,1% p/v de mono- et triesters de polyoxyéthylène sorbitane, de chlorure de benzalkonium, de cholate de sodium, de taurocholate de sodium, de désoxycholate de sodium ou de glycocholate de sodium; et 0,001 à 0,005 M d'acide éthylène-diamine-tétra-acétique, d'acide citrique, d'éthylène-bis-oxyéthylène-nitrile ou d'orthophénanthrolène.